# FASCICULE DE BREVET EUROPEEN

(11) **EP 1 462 180 B1**
(45) Date de publication et mention de la délivrance du brevet: **11.03.2009**
(21) Numéro de dépôt: 03292971.3
(22) Date de dépôt: 28.08.2001
(51) Int. Cl.: B05B 11/00, A61M 15/00

(54) **Dispositif de distribution de produit fluide de type multidose**
Vorrichtung zur wiederholten Abgabe von Mediendosen
Multiple-dose device for dispensing a fluid product

(30) Priorité: 07.09.2000 FR 0011429
(43) Date de publication de la demande: 29.09.2004
(62) Demande divisionnaire de: 01965366.6
(73) Titulaire: Valois SAS, 27110 Le Neubourg (FR)
(72) Inventeur: Stradella, Giuseppe, 16032 Camogli (GE) (IT)
(74) Mandataire: CAPRI

(56) Documents cités:
- WO-A-98/12511
- US-A- 4 771 769
- PATENT ABSTRACTS OF JAPAN vol. 1998, no. 12, 31 octobre 1998 (1998-10-31) -& JP 10 179739 A (TAISEI KAKO KK), 7 juillet 1998 (1998-07-07)

## Description

La présente invention concerne un dispositif de distribution de produit fluide, et plus particulièrement un dispositif de pulvérisation de produit fluide du type multidose comprenant un actionnement latéral.

Dans le domaine des dispositifs de pulvérisation multidose, plusieurs systèmes pour réaliser un actionnement latéral de la pompe du dispositif ont été développés récemment.

Ces systèmes sont généralement constitués d'un corps contenant l'ensemble du dispositif de pulvérisation et pourvu d'un système de levier pivotant agissant contre le fond du réservoir de produit et le poussant axialement, pendant l'actionnement, en direction de la tête de distribution de telle manière à reproduire le mouvement de la main pendant un actionnement axial standard.

Ces systèmes présentent de nombreux problèmes. En particulier, ils ne permettent pas un procédé d'assemblage standard au niveau du fabricant du produit à distribuer, généralement un produit pharmaceutique, puisqu'il n'est alors pas possible de simplement introduire l'unité formée par le réservoir et la pompe dans la tête de distribution, comme cela est habituel avec les dispositifs ayant un actionnement manuel axial. De plus, ces systèmes rendent l'actionnement latéral obligatoire, alors que l'utilisateur peut pour diverses raisons, telles que des problèmes de manipulation, une habitude difficile à modifier, etc., parfois préférer pouvoir actionner son dispositif également de la manière traditionnelle, c'est-à-dire en appuyant manuellement sur le fond du réservoir. De plus, tous les systèmes d'actionnement latéral existant ne résolvent pas le problème qui est typique avec la plupart des pompes de pulvérisation, et qui concerne la dépendance en terme de dosage et de qualité de spray de la vitesse et de la force de l'actionnement prévu par l'utilisateur. Ainsi, un actionnement partiel peut engendrer la distribution d'une dose partielle, ou altérer la qualité du spray.

Le document JP 10179739 décrit un système d'actionnement qui coopère avec le fond du réservoir.

La présente invention a pour but de fournir un dispositif de distribution de produit fluide qui ne reproduit pas les inconvénients susmentionnés.

En particulier, la présente invention a pour but de fournir un dispositif de distribution de produit fluide qui soit simple et peu coûteux à fabriquer et à assembler.

La présente invention a également pour but de fournir un dispositif de distribution de produit fluide qui garanti une pulvérisation parfaite et un dosage complet à chaque actionnement, indépendamment de la résistance de la pompe et/ou de la quantité de produit et/ou de la force d'actionnement exercé par l'utilisateur sur l'élément d'actionnement.

La présente invention a également pour but de fournir un dispositif de distribution de produit fluide qui peut être actionné par un dispositif d'actionnement latéral ou par un actionnement standard, c'est-à-dire une poussée axiale exercée sur le fond du réservoir en direction de l'orifice de distribution.

La présente invention a également pour but de fournir un dispositif de distribution de produit fluide à actionnement latéral dans lequel l'assemblage du dispositif au niveau du fabricant du produit n'est pas modifié par la présence du système d'actionnement latéral, c'est-à-dire qu'après remplissage du réservoir, l'unité formée du réservoir et de l'organe de distribution est simplement fixé à l'intérieur du reste du dispositif.

La présente invention a donc pour objet un dispositif de distribution de produit fluide tel que décrit dans la revendication 1.

Des caractéristiques avantageuses sont décrites dans les revendications dépendantes.

D'autres caractéristiques et avantages de la présente invention apparaîtront plus clairement au cours de la description détaillée suivante de deux modes de réalisation de l'invention, en référence aux dessins joints, donnés à titres d'exemples non limitatifs, sur lesquels :
- La figure 1 est une vue schématique en section transversale partiellement découpée d'un dispositif de distribution de produit fluide selon un premier mode de réalisation de la présente invention, en position de repos,
- La figure 2 est une vue similaire à celle de la figure 1, en position d'actionnement,
- La figure 3 est une vue schématique en section transversale partiellement découpée d'un second mode de réalisation de l'invention, en position de repos,
- La figure 4 est une vue similaire à celle de la figure 3, en position d'actionnement,
- La figure 5 est une vue schématique en section horizontale vue du dessus du dispositif représenté sur la figure 3, en position de repos, et
- La figure 6 est une vue similaire à celle de la figure 5 en position d'actionnement.

En référence aux dessins, le dispositif selon l'invention comporte un réservoir 3 sur lequel est fixé un organe de distribution (non représenté) telle qu'une pompe ou une valve doseuse. Les exemples représentés sur les dessins concernent un dispositif comportant une pompe qui fonctionne avec un piston se déplaçant à l'intérieur d'une chambre de dosage pour expulser une dose de produit, comme cela est bien connu, mais l'invention s'applique également aux inhalateurs du type MDI, comportant une valve doseuse, et généralement utilisés en position inversée. Dans ce cas, c'est la soupape de la valve qui se déplace par rapport au réservoir pour expulser la dose.

L'organe de distribution, que l'on désignera ci-après par le terme pompe, est monté sur le réservoir 3 de préférence au moyen d'une bague de fixation 8. L'unité formé par le réservoir et la pompe est insérée dans un corps 1 qui incorpore un embout nasal 2 pourvu d'un orifice de distribution. Bien que représentée dans les exemples sous la forme d'un dispositif de type nasal, l'invention pourrait également s'appliquer à d'autres types de dispositifs de distribution, par exemple les inhalateurs du type buccal ou similaire.

Selon l'invention, le dispositif comporte un élément d'actionnement 5 qui est adapté à actionner la pompe pour expulser une dose de produit. Cet élément d'actionnement 5 est disposé latéralement, et déplaçable dans une direction différente, et notamment environ perpendiculaire à la direction de déplacement axiale du piston dans la pompe. Ainsi, l'élément d'actionnement 5 comporte une partie d'extrémité pourvue d'une surface de came 6 qui coopère avec une ou plusieurs projections 7 prévu sur la bague de fixation 8. Dans les exemples représentés, ces projections radiales 7 sont réalisées sous forme de paire qui sont solidaires de la bague de fixation 8. La surface de came de l'élément d'actionnement 5 est avantageusement réalisée sous forme de pente inclinée 6 qui coopère avec lesdites projections 7 pour transformer un déplacement environ radial de l'élément d'actionnement 5 en un déplacement axial du piston de la pompe, pour distribuer une dose de produit. Lorsqu'on relâche la pression sur l'élément d'actionnement 5, celui-ci est ramené vers sa position de repos par le ressort de rappel du piston.

Le dispositif peut comporter des moyens de régulation de force qui sont adaptés à prédéterminer la force exercée par l'élément d'actionnement 5 sur lesdites projections 7, indépendamment de la résistance de la pompe et/ou de la quantité de liquide dans le réservoir et/ou de la force d'actionnement exercée par l'utilisateur sur l'élément d'actionnement 5.

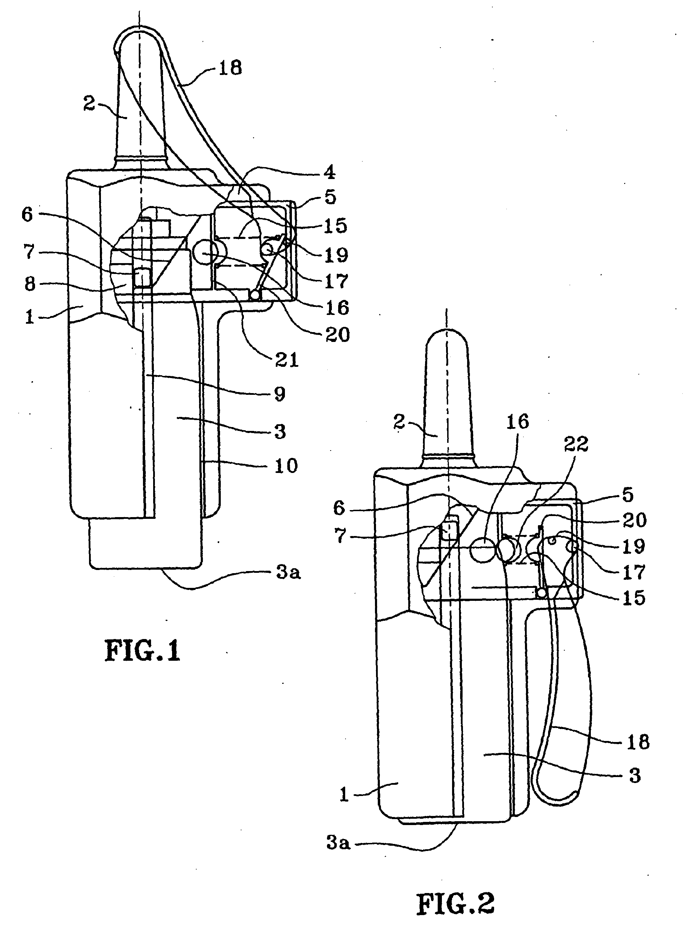

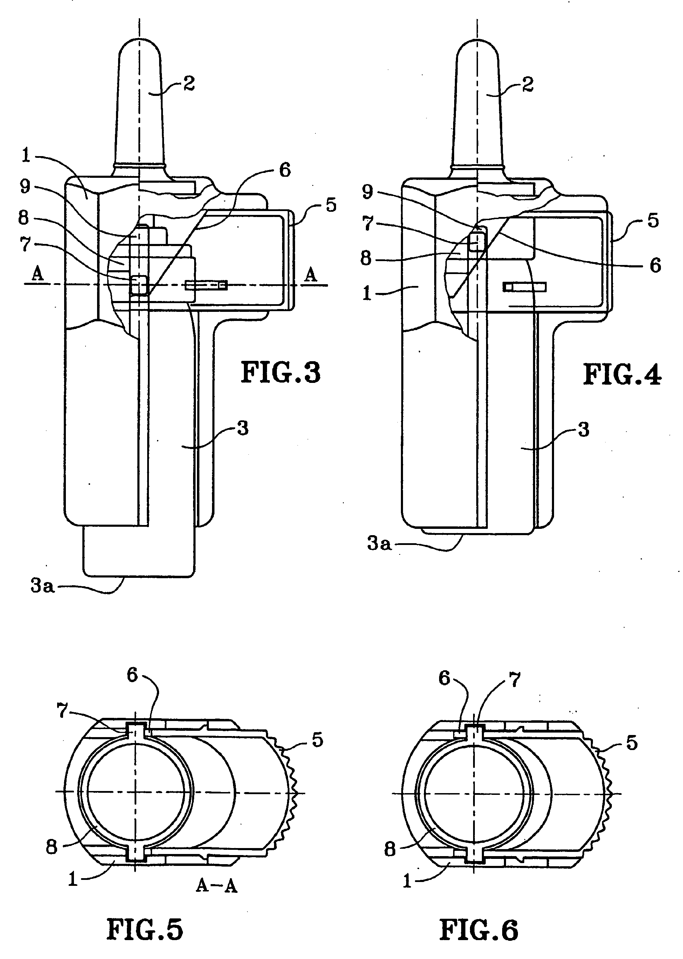

Selon un premier mode de réalisation sur les figures 1 et 2, ces moyens de régulation de force comprennent des moyens élastiques tel qu'un ressort 15, qui coopère avec l'élément d'actionnement 5 pour le solliciter vers sa position d'actionnement lorsque le ressort est comprimé. Les moyens de régulations de force comportent en outre des moyens de déclenchement 16, de préférence réalisés sous la forme d'un ou deux boutons latéraux. Ces boutons 16 retiennent l'élément d'actionnement en position de repos, représentée sur la figure 5, contre la force exercée par le ressort 15, jusqu'à ce que l'utilisateur appuie sur les boutons 16 pour libérer le blocage de l'élément d'actionnement 5 et ainsi permettre son déplacement vers sa position d'actionnement sous l'effet de la force élastique du ressort 15, et ainsi déplacer le réservoir 3 par rapport au piston de la pompe pour expulser une dose. C'est donc les caractéristiques du ressort 15 qui déterminent la force exercée par l'élément d'actionnement sur la pompe, et par conséquent la force d'actionnement sur la pompe est indépendante de la force exercée par l'utilisateur sur l'élément d'actionnement 5. Avantageusement, le ressort 15 coopère avec un élément de chargement 18 qui est déplaçable manuellement pour comprimer ledit ressort 15. Comme représenté sur les dessins, cet élément de chargement 18 peut être réalisé sous la forme d'un capot, qui en position de repos, obture l'orifice de distribution du dispositif, et qui est monté pivotant sur le corps 1 autour d'un axe de pivotement 17, ledit capot 18 comportant en outre une cheville de commande transversale 19 qui coopère avec un levier 20. Lorsqu'on ouvre le capot 18, comme représenté sur la figure 2, la cheville 19 agit contre le levier 20 qui comprime le ressort 15 contre une paroi interne 21 formée à l'intérieur de l'élément d'actionnement 5. Dans cette position, l'élément d'actionnement 5 est maintenue dans sa position de repos par la paire de boutons 16. De préférence, ces boutons 16 sont formés sur des bras flexibles (non représentés) solidaires de l'élément d'actionnement 5, et qui en position de repos de l'élément d'actionnement 5 sont sollicités élastiquement dans des trous 22 du corps 1. Une pression latérale appliquée par l'utilisateur sur les boutons 16 qui sont formés de manière appropriée, leur permet de sortir des trous de retenue 22 et par conséquent de libérer l'élément d'actionnement 5, qui sous la poussée du ressort 15 glisse à l'intérieur du manchon 4 du corps 1 pour actionner la pompe. Il est à noter que pendant ce déplacement de l'élément d'actionnement, l'axe de pivotement 17 du capot 18 sur le corps 1 n'empêche pas le déplacement de l'élément d'actionnement 5 puisque ledit capot est pourvu de deux fentes appropriées (non représentées) sur ces côtés pour empêcher toute interférence avec lesdites chevilles formant l'axe de pivotement 17.

Eventuellement, l'élément d'actionnement peut comporter en outre des moyens de rappel élastiques, qui ramènent l'élément d'actionnement dans sa position de repos après chaque actionnement du dispositif. Généralement, toutefois, la force du ressort de rappel du piston de la pompe suffit pour ramener l'élément d'actionnement 5 vers sa position de repos, par l'intermédiaire des projections 7 poussant sur le plan incliné 6. De préférence, lorsque l'élément d'actionnement 5 revient vers sa position de repos, les boutons 16 reviennent automatiquement dans les trous 22 du corps, et le dispositif est prêt pour être réutilisé.

Si souhaité, ces moyens de rappel de l'élément d'actionnement 5 vers sa position de repos peuvent être actionnés uniquement lorsque le capot 18 est ramené vers sa position d'obturation de l'orifice de distribution représenté sur la figure 1. Dans ce cas, on garantit que l'utilisateur est obligé de refermer le capot, et ainsi de protéger l'orifice de distribution du dispositif après chaque utilisation.

Dans les figures 3 à 6, il est représenté un second mode de réalisation qui diffère de celui représenté sur les figures 1 et 2 en ce que le dispositif ne comporte pas les moyens élastiques précomprimables pour exercer une force sur l'élément d'actionnement 5 lors de l'actionnement du dispositif. Dans l'exemple représenté sur les figures 3 à 6, la force exercée par l'utilisateur sur l'élément d'actionnement latéral 5 est directement transformé en force de déplacement axial du piston de la pompe par la pente inclinée 6 formant la surface de came et coopérant avec la cheville 7 solidaire de la bague de fixation 8. Dans ce mode de réalisation, les moyens de régulation de force peuvent être réalisés sous la forme d'une pente variable dudit plan incliné 6. En effet, les pompes changent généralement leur résistance, et par conséquent la force requise pour l'actionnement, pendant la course d'actionnement. Ces augmentations de résistance pendant la course se produisent parfois de manière constante, parfois très rapidement. Une inclinaison de la surface de came 6 avec une variation appropriée peut totalement compenser ces augmentations de résistance et ainsi permettrent un actionnement sûr, avec la conséquence d'un dosage parfait et d'une pulvérisation parfaite, tout en exerçant une force constante sur l'élément d'actionnement 5.

Le dispositif selon l'invention présente donc les avantages suivants :
- l'actionnement est latéral, et par conséquent souvent plus facile et plus confortable pour l'utilisateur ;
- l'ensemble du système d'actionnement latéral est assemblé sur le corps 1, à proximité de l'embout nasal 2 du dispositif, de sorte que le fond du réservoir 3 peut être librement accessible. Ceci permet un actionnement traditionnel standard par appui axial sur le fond du réservoir si nécessaire. Ceci peut être déterminant en cas de crise, par exemple de crise d'asthme, lorsque l'utilisateur, dans sa panique, essaie d'actionner le dispositif dans la manière dont il en a l'habitude depuis de nombreuses années, c'est à dire par une poussée sur le fond du réservoir.
- l'assemblage au niveau du fabricant du produit à distribuer n'est pas modifié par la présence d'un actionnement latéral, l'unité formée du réservoir et de la pompe pouvant être simplement montée dans le corps comme cela est réalisé habituellement avec les dispositifs existants aujourd'hui. L'actionnement latéral ne modifie dans ce cas pas l'assemblage du dispositif, ce qui est un avantage considérable.
- le système d'actionnement latéral étant indépendant de la dimension du réservoir, la présente invention permet d'adapter des réservoirs de dimensions différentes à l'intérieur du même corps 1 sans avoir à modifier la chaîne de production du dispositif.

Bien que représenté en relation avec une pompe comportant un piston et fonctionnant en position droite sur les dessins, l'invention, comme précisé précédemment, s'applique également à des valves doseuses pour des inhalateurs du type MDI. D'autre part, l'invention n'est pas limitée aux exemples de réalisation représentés sur les figures, et des modifications sont envisageables dans le cadre de l'invention qui est défini par les revendications annexées.

## Revendications

1. Dispositif de distribution de produit fluide, comprenant un corps (1), un réservoir de produit (3), un organe de distribution, tel qu'une pompe ou une valve doseuse comportant un piston ou une soupape déplaçable axialement, monté sur le réservoir (3), et un élément d'actionnement (5), déplaçable entre une position de repos et une position d'actionnement, pour actionner l'organe de distribution et ainsi distribuer sélectivement le produit contenu dans le réservoir (3), **caractérisé en ce que** la direction de déplacement dudit élément d'actionnement (5) est différente, notamment environ perpendiculaire, à la direction de déplacement axiale du piston ou de la soupape de l'organe de distribution, l'élément d'actionnement (5) comportant une partie d'extrémité pourvue d'une surface de came (6) qui coopère avec une bague de fixation (8) adaptée à fixer l'organe de distribution sur le réservoir (3), de sorte qu'un déplacement environ radial de l'élément d'actionnement (5) vers sa position d'actionnement engendre un déplacement axial du réservoir (3) par rapport au piston ou à la soupape de l'organe de distribution pour distribuer une dose de produit.

2. Dispositif selon la revendication 1, dans lequel ladite surface de came comporte un plan incliné (6) coopérant avec au moins une projection (7) solidaire de la bague de fixation (8).

3. Dispositif selon la revendication précédente 1 ou 2, dans lequel l'élément d'actionnement (5) comporte des moyens de rappel le ramenant de sa position d'actionnement à sa position de repos après chaque actionnement.

4. Dispositif selon l'une quelconque des revendications précédentes, dans lequel le réservoir (3) et ledit organe de distribution forment une première unité, ledit corps (1) et ledit élément d'actionnement (5) formant une seconde unité, ladite première unité étant fixée, notamment encliquetée, dans ladite seconde unité.

5. Dispositif selon la revendication 4, dans lequel, après fixation de la première unité dans la deuxième unité, le fond (3a) du réservoir (3) reste accessible pour un actionnement manuel par poussée axiale sur ledit fond du réservoir.

6. Dispositif selon l'une quelconque des revendications précédentes, dans lequel ledit organe de distribution est une pompe comportant un piston.

7. Dispositif selon l'une quelconque des revendications 1 à 5, dans lequel ledit organe de distribution est une valve doseuse comportant une soupape.

## Claims

1. A fluid dispenser device comprising a body (1), a fluid reservoir (3), a dispensing member, such as a pump or a metering valve having a piston or a valve member that is mounted to move axially, which dispensing member is fitted to the reservoir (3), and an actuating element (5) mounted to move between a rest position and an actuating position, for actuating the dispensing member and thus for selectively dispensing the fluid contained in the reservoir (3), said fluid dispenser device being **characterized in that** the direction in which said actuating element (5) moves is different from, and in particular perpendicular to, the axial direction in which the piston or the valve member of the dispensing member moves, the actuating element (5) having an end portion provided with a cam surface (6) which co-operates with a fixing ring (8) adapted to fix the dispensing member to the reservoir (3) so that the actuating element (5) being moved substantially radially into its actuating position causes the reservoir (3) to be moved axially relative to the piston or to the valve member of the dispensing member so as to dispense a dose of fluid.

2. A device according to claim 1, in which said cam surface comprises a sloping surface (6) co-operating with at least one projection (7) integral with the fixing ring (8).

3. A device according to claim 1 or 2, in which said actuating element (5) is provided with return means for returning it from its actuating position to its rest position after each occasion on which the device is actuated.

4. A device according to any preceding claim, in which the reservoir (3) and said dispensing member form a first unit, said body (1) and said actuating element (5) forming a second unit, said first unit being fixed, in particular by snap-fastening, into said second unit.

5. A device according to claim 4, in which, after fixing of said first unit in said second unit, the end-wall (3a) of the reservoir (3) remains accessible for being actuated by hand by pushing axially on said end-wall of the reservoir.

6. A device according to any preceding claim, in which said dispensing member is a pump including a piston.

7. A device according to any one of claims 1 to 5, in which said dispensing member is a metering valve including a valve member.

## Patentansprüche

1. Vorrichtung zur Fluidproduktabgabe, aufweisend einen Körper (1), einen Produktvorratsbehälter (3), ein Abgabeorgan, wie etwa eine Pumpe, oder ein Dosierventil, aufweisend einen Kolben, oder ein Ventilorgan, der bzw. das axial verschiebbar ist und auf dem Vorratsbehälter (3) angebracht ist, und ein Betätigungselement (5), welches zwischen einer Ruhestellung und einer Betätigungsstellung verschiebbar ist, um das Abgabeorgan zu betätigen und **dadurch** selektiv das im Vorratsbehälter (3) enthaltene Produkt abzugeben, **dadurch gekennzeichnet, dass** die Verschiebungsrichtung des Betätigungselements (5) der axialen Verschiebungsrichtung des Kolbens oder des Ventilorgans des Abgabeorgans unterschiedlich - in etwa lotrecht dazu verlaufend - ist, wobei das Betätigungselement (5) einen Endteil aufweist, der mit einer Nockenfläche (6) versehen ist, die mit einem Festlegungsring (8) zusammenwirkt, der dazu ausgelegt ist, das Abgabeorgan auf dem Vorratsbehälter festzusetzen, so dass eine in etwa radiale Verschiebung des Betätigungselements (5) in seine Betätigungsstellung mit einer axialen Verschiebung des Vorratsbehälters (3) in Bezug auf den Kolben oder das Ventil des Abgabeorgans einhergeht, um eine Produktdosis abzugeben.

2. Vorrichtung nach Anspruch 1, wobei die Nockenfläche eine geneigte Ebene (6) aufweist, welche mit zumindest einem Vorsprung (7) zusammenwirkt, der mit dem Festlegungsring (8) fest verbunden ist.

3. Vorrichtung nach Anspruch 1 oder 2, wobei das Betätigungselement (5) Rückstellmittel aufweist, welche sie in die Betätigungsstellung ausgehend von ihrer Ruhestellung nach jeder Betätigung rückstellt.

4. Vorrichtung nach einem der vorangehenden Ansprüche, wobei der Vorratsbehälter (3) und das Abgabeorgan eine erste Einheit bilden, wobei der Körper (1) und das Betätigungselement (5) eine zweite Einheit bilden, wobei die erste Einheit in der zweiten Einheit stationär, insbesondere verrastet ist.

5. Vorrichtung nach Anspruch 4, wobei nach der Festlegung der ersten Einheit in der zweiten Einheit der Boden (3a) des Vorratsbehälters (3) für eine manuelle Betätigung durch axiales Drücken auf den Boden des Vorratsbehälters zugänglich bleibt.

6. Vorrichtung nach einem der vorangehenden Ansprüche, wobei das Abgabeorgan eine Pumpe ist, die einen Kolben aufweist.

7. Vorrichtung nach einem der Ansprüche 1 bis 5, wobei das Abgabeorgan ein Dosierventil ist, welches ein Ventilorgan aufweist.
